# EUROPEAN PATENT SPECIFICATION

(11) **EP 1 935 946 B1**
(45) Date of publication and mention of the grant of the patent: **01.02.2012**
(21) Application number: 07023303.6
(22) Date of filing: 30.11.2007
(51) Int. Cl.: C09C 1/00

(54) **Filler, process for producing the same, and cosmetic**
Füller, Herstellungsverfahren dafür und Kosmetikprodukt
Agent de remplissage, son procédé de production et composition cosmétique

(30) Priority: 22.12.2006 JP 2006345638
(43) Date of publication of application: 25.06.2008
(73) Proprietor: Merck Patent GmbH, 64293 Darmstadt (DE)
(72) Inventor: Noguchi, Tamio, Dr., Iwaki-shi Fukushima Ken 972-8322 (JP); Watanabe, Yukitaka, Iwaki-shi Fukushima Ken 970-8035 (JP); Sasaki, Fumiko, Iwaki-shi Fukushima Ken 971-8184 (JP)

(56) References cited:
- EP-A2- 1 059 338
- DE-A1- 19 618 569
- US-A- 4 494 993

## Description

### [Technical Field]

The present invention relates to a filler used in a variety of applications. Specifically, the present invention relates to a filler for a cosmetic, particularly suitable to use in a foundation.

### [Backround Art]

Among make-up cosmetics, foundations applied to skin have constantly been demanded to have a comfortable and favourable touch and good adherence to skin and last longer (Material Technology (ZAIRYO GIJYUTSU in Japanese), 16 (2), 64 (1998)). Thin platelet-like mica is used in foundations because it can have good extensibility and adhesion and produce comfort (Journal of the Society of Powder Technology, Japan, 21, (9), 565, (1984)). However, the use of mica alone is not preferable because mica, when mixed with oil, exhibits unnatural luster. To reduce this luster, the present inventors have proposed a filler for a foundation comprising mica coated on its surface with a fine particle of a metallic oxide and having a so-called soft focus characteristic where a light-scattering property on the surface is enhanced. For example, Japanese Patent Publication No. H2-42388 (Patent Document 1) and Japanese Patent Laid-Open No. H5-287212 (Patent Document 2) have disclosed a filler comprising mixa coated on its surface with particles of barium sulphate with fine aggregated titanium oxide and particles of titanium oxide. Japanese Patent Laid-Open No. 2001-098186 (Patent Document 4) has disclosed a filler comprising mica coated with spherical particulate silicon oxide and particles of titanium oxide.

In the latest fashion, foundations that have transparency serving as a factor of beautiful skin, improve skin tone, accomplish more natural make-up, and have the higher effect of masking wrinkles have been demanded in order to produce healthy beauty (see e.g., J. Soc. Cosmet. Chem. Jpn. 39 (3), 201-208, (2005) (Non-patent Document1 ); Skin and Beauty (HIFU TO BIYO in Japanese), 124 (4), 4080 (1992) (Non-patent Document 2); and Surface (HYOMEN in Japanese), 30(9), 703 (1992) (Non-patent Document 3)). That is to say, as optical functions of foundations, there have been demanded properties that is capable of covering irregular skin color, wrinkles, and the like, with a thin film and giving a light and natural finish. Moreover, foundations are applied to skin and therefore, of course, required to have good feel of use.

A variety of fillers to be contained in foundations have been studied so far in order to enhance the functionality of foundations. Particularly, titanium oxide-coated mica, a pearl iridescent pigment having interference color, has received attention in that it can cover irregular skin color and wrinkles by its transmitted light. However, the titanium oxide-coated mica generates unnatural luster to skin. To reduce the luster, it has been known that barium sulfate or fine particles of polymer for light scattering are dispersedly deposited onto the titanium oxide-coated mica (FRAGRANCE JOURNAL, 34 (2) 67 (2006) (Non-patent Document 4) and FRAGRANCE JOURNAL, 28 (5) 13 (2000) (Non-patent Document 5)). However, these fillers are merely intended for light scattering on the surface and, hence, do not have transparent nature and cause problem of a whitish or powdery finish in facial make-up.

Fillers having better transparent nature have been proposed, which include an inorganic composite powder comprising two or more types of inorganic oxides with different refractive indexes respectively and sequentially laminated from the one with the largest refractive index at the bottom on a thin platelet-like substrate such as mica (WO99/49834 (Patent Document 5)), a laminate-type interference spherical pigment comprising a core-shell structure where a metallic oxide with a low refractive index and a metallic oxide with a high refractive index are layered alternately (Japanese Patent Laid-Open No. 2003-55573 (Patent Document 6)), a UV ray-blocking composite material comprising mica coated with silica having a low refractive index and titanium oxide having a high refractive index alternately (J. Soc. Cosmet. Chem. Jpn. 40 (1) 34 (2006) (Non-patent Document 6)), and a filler coated on its surface with a semitransparent layer such as a filler where a thin platelet-like substrate such as mica is coated with iron oxide and then coated with silica (FRAGRANCE JOURNAL, 34 (2) 74 (2006) (Non-patent Document 7)).
[Patent Document 1] Japanese Patent Publication No. H2-42388
[Patent Document 2] Japanese Patent Laid-Open No. H5-287212
[Patent Document 3] Japanese Patent Publication No. H8-13943
[Patent Document 4] Japanese Patent Laid-Open No. 2001-098186
[Patent Document 5] WO99/49834
[Patent Document 6] Japanese Patent Laid-Open No. 2003-55573
[Non-patent Document 1] J. Soc. Cosmet. Chem. Jpn. 39 (3), 201-208, (2005)
[Non-patent Document 2] Skin and Beauty (HIFU TO BIYO in Japanese), 124 (4), 4080 (1992)
[Non-patent Document 3] Surface (HYOMEN in Japanese), 30 (9), 703 (1992)
[Non-patent Document 4] FRAGRANCE JOURNAL, 34 (2) 67 (2006)
[Non-patent Document 5] FRAGRANCE JOURNAL, 28 (5) 13 (2000)
[Non-patent Document 6] J. Soc. Cosmet. Chem. Jpn. 40 (1) 34 (2006)
[Non-patent Document 7] FRAGRANCE JOURNAL, 34 (2) 74 (2006)

### [Disclosure of the Invention]

### [Problems to be Solved by the Invention]

However, no conventional cosmetic fillers, simultaneously with good balance, satisfied covering ability for sufficiently cover irregular skin color, blotches, pigmented spots, wrinkles, pores, and the like, natural beautiful skin with transparency for giving a healthy look, light skin tone, and good feeling of use. Particularly, a material having transparency while having a moderate light-scattering property and sufficient covering ability, that is, a filler exhibiting so-called foggy effects excellent in a moderate scattering property against transmitted light, as in fogged glasses, has not been found yet.

An object of the present invention is to provide a filler capable of exhibiting semitransparent foggy effects as in fogged glasses.

### [Means for Solving the Problems]

The present invention relates to the following items.
1. A filler comprising:
   a transparent or semitransparent thin platelet-like substrate;
   a particle layer or coating layer (hereinafter, referred to as a first layer) which is formed on the surface of the thin platelet-like substrate and contains a hydrous oxide, carbonate, or calcined product thereof, of at least one metal (hereinafter, referred to as a first metal) selected from the group consisting of Mg, Al, and Ca;
   a coating layer (hereinafter, referred to as a second layer) which coats the thin platelet-like substrate comprising the first layer formed thereon and contains an Si hydrous oxide or calcined product thereof; and a coating layer (hereinafter, referred to as a third layer) which coats the second layer and contains a Ti hydrous oxide or calcined product thereof.
2. A filler according to above item 1, wherein the thin platelet-like substrate has a shape with an aspect ratio of 10 to 100 represented by average particle diameter/average thickness and an average particle diameter of 5 to 20 µm.
3. A filler according to above item 1 or 2, wherein the thin platelet like substrate is selected from the group consisting of, natural and synthetic mica, thin platelet-like alumina, talc, glass flake, kaoline and thin platelet-like silica.
4. A filler according to any of above items 1 to 3, wherein an interface between the second layer and the third layer has, in a range of 1 µm distance, the asperity of second layer where a difference between the highest and lowest heights from the substrate surface ranges from 30 nm to 200 nm.
5. A filler according to any of above items 1 to4, wherein the filler comprises aggregates of 70 nm to 500 nm formed of crystals of the Ti hydrous oxide or calcined product thereof on the surface.
6. A filler according to any of above items 1 to 5, wherein the proportion by weight of each layer to the whole filler is
   1 to 7% by weight of the first layer in terms of a metallic oxide of the first metal,
   10 to 40% by weight of the second layer in terms of silicon dioxide, and
   20 to 50% by weight of the third layer in terms of titanium dioxide.
7. A filler according to any of above items 1 to 6, wherein a dried coating film including 20% by weight of the filler and having a film thickness ranging from 7.0 to 15 µm exhibits perfect luminous transmittance (τt) of 70% or higher, parallel light transmittance of 20% or higher each according to ISO 13468-1 (JIS K7361), and haze of 40 to 70 according to ISO 14782 (JIS K7136).
8. A filler according to any of above items 1 to 7, wherein the filler has oil absorption ranging from 80 to 150 ml/100 g.
9. A filler according to any of above items 1 to 8, wherein the filler has a friction coefficient (MIU value) of 0.75 or lower measured with a KES friction tester.
10. A process for producing a filler, comprising:
   a first layer-forming step of suspending a transparent or semitransparent thin platelet-like substrate in water and gradually adding to this suspension under neutral or basic conditions, an aqueous solution of a salt of at least one metal (hereinafter, referred to as a first metal) selected from the group consisting of Mg, Al, and Ca and an alkali aqueous solution or carbonate aqueous solution, thereby depositing a hydrous oxide and/or carbonate of the first metal in a particle or layer form onto the surface of the thin platelet-like substrate;
   a second layer-forming step of gradually adding an alkali aqueous solution of silicic acid and dilute mineral acid at the same time under neutral or basic conditions, thereby coating with an Si hydrous oxide, the thin platelet-like substrate comprising the first layer formed thereon; and
   a third layer-forming step of gradually adding a Ti salt aqueous solution and an alkali aqueous solution at the same time under acidic conditions, thereby coating with a Ti hydrous oxide, the thin platelet-like substrate comprising the second layer formed thereon.
11. A process according to above item 10, further comprising a step of calcining the thin platelet-like substrate after the formation of the third layer.
12. Use of the filler according to any of above items 1 to 9 in cosmetics, lacquers, plastics, inks, printing inks and coatings.
13. A cosmetic containing 1-50 wt.% based on the cosmetic formulation of a filler according to any of above items 1 to 9.

### [Best Mode for Carrying Out the Invention]

Hereinafter, a filler of the present invention will be described along with a production process of the present invention.

A thin platelet-like substrate used in the filler may be any of transparent or semitransparent substances. A low lustrous substance is particularly preferable for uses in cosmetics. Specifically, natural mica such as muscovite and sericite, synthetic mica, thin platelet-like alumina, talc, glass flake, kaoline and thin platelet-like silica, and the like are preferable.

Among them, micas such as muscovite and sericite, which are inexpensive and are highly stably supplied are preferably recommended.
The particle size of the thin platelet-like substrate is preferably 5 to 20 µm in average particle diameter. If the average particle diameter is too small, required semi-transparency is difficult to obtain due to aggregation. If the average particle diameter is too large, favorable texture to skin is difficult to obtain. The aspect ratio (average particle diameter/average thickness) of the thin platelet-like substrate is 10 to 100, preferably 20 to 50. A too small aspect ratio (too large thickness) is not preferable because a covering property is increased (transparency is reduced). On the other hand, a too large aspect ratio provides too high transparency and presents the problem that the mechanical strength of the particle itself is not maintained. Regarding the particle size, "average" means volume average and measured by, for example, Mastersizer-2000 manufactured by Malvern Instruments Ltd.

In a first layer-forming step, a hydrous oxide and/or carbonate of at least one metal (first metal) selected from the group consisting of Mg, Al, and Ca are deposited onto the surface of the thin platelet-like substrate. Specifically, the thin platelet-like substrate particle is suspended in water, and to this suspension, an aqueous solution of a salt of the first metal is gradually added, for example, by dropping, usually with stirring. At the same time, an alkali aqueous solution or carbonate aqueous solution is gradually added, for example, by dropping, to thereby deposit the first metal in hydrous oxide and/or carbonate forms onto the substrate surface. In this context, the phrase "gradually added" means gradually added to the extent that allows the almost whole amount of the hydrous oxide and/or carbonate of the first metal added to deposit onto the thin platelet-like substrate. The same holds true for second and third layers described below. The layers deposited on the surface means that the substrate is completely covered by first layer and does not contain any open edges. The second layer and the third layer each completely cover the coated substrate.

The salt of the first metal may be a water-soluble salt. Examples of the Mg salt include magnesium chloride, magnesium sulfate, and magnesium nitrate. Examples of the Al salt include aluminum chloride, aluminum sulfate, aluminum nitrate, sodium aluminate, and potassium aluminate. Examples of the Ca salt include calcium chloride and calcium nitrate. These salts may be mixed, or alternatively, different salts of the same metal or salts of different metals may be used. The thin platelet-like substrate is coated with hydrous oxides and/or carbonates comprising two or more metals, or in the form of composite metal by using the salts of different metals. Al is particularly preferable as the first metal. Thus, the Al salt is preferably employed.

Simultaneously with the addition of the aqueous solution of the salt of the first metal, an alkali aqueous solution or carbonate aqueous solution can be added to the suspension of the thin platelet-like substrate particle to thereby deposit the hydrous oxide or carbonate of the first metal onto the substrate surface. To convert the first metal to the hydrous oxide form, an alkali compound is used. For example, sodium hydroxide or potassium hydroxide is preferable. To convert the first metal to the carbonate form, sodium carbonate, potassium carbonate, or the like is used. In general, they are used in an aqueous solution form.

For the addition of the aqueous solution of the salt of the first metal and the alkali aqueous solution or carbonate aqueous solution, it is preferred that conditions such as pH in the suspension, a temperature, and addition time should be set so as to attain the intended purposes. In general, the suspension is maintained under neutral or basic conditions, particularly under weak basic conditions, for example, at pH ranging from 5 to 10, particularly preferably 6 to 9. The temperature can be set appropriately and ranges from, for example, room temperature to 100°C, preferably 40 to 90°C. The whole amount of the first metal added can be deposited onto the substrate surface by properly selecting the conditions.

In the present invention, the first layer comprising the hydrous oxide and/or carbonate of the first metal deposited thereto is preferably 1 to 7% by weight in terms of its metallic oxide weight with respect to the whole weight of the filler (which means the total sum of the substrate weight and all the first to third layer weights in terms of their metallic oxide weights, the same holds true for descriptions below). The amount of its usage may be large for the thin platelet-like substrate used having a large surface area (small average particle diameter), while the amount of its usage may be small for the thin platelet-like substrate having a large particle diameter.

The amount of the deposition the first layer has a great influence on the performance of the filler of the present invention. Preferably, a second layer of an Si hydrous oxide has asperity on its surface, as described later. The amount of the deposition of the first layer has an influence on the asperity on the surface of the second layer. If the first layer is absent or deposited in a too small amount, the coating layer of the Si hydrous oxide cannot be formed on the thin platelet-like substrate. On the other hand, if the amount of the first layer is too large, the thickness of the coating layer is increased, with the result that the coating layer of the Si hydrous oxide has a flat and smooth surface, which is less likely to become asperate.

Thus, the amount of the first layer deposited is particularly preferably 2 to 7% by weight, most preferably 2.5 to 6% by weight. The first layer is deposited in a form of particles or in a form of layer onto the surface of the thin platelet-like substrate. When the amount of the first layer, albeit in the layer form, falls within the range described above, asperity is formed on the surface of the second layer of the Si hydrous oxide. The term "in a form of particles or in a form of layer" used herein is defined to mean any intermediate states between "particles" and "continuous layer" in addition to "particles" and "continuous layer".

In the subsequent second layer-forming step, the thin platelet-like substrates comprising the first layer formed thereon are coated with the Si hydrous oxide. An alkali compound of silicic acid for forming the Si hydrous oxide layer is selected from sodium silicate, potassium silicate, and the like. Preferably, they are used in an aqueous solution form. Examples of mineral acid added simultaneously therewith (preferably, by dropping) include hydrochloric acid, sulfuric acid, and nitric acid. Preferably, they are respectively diluted with water and used.

Conditions such as pH in the suspension, a temperature, and addition time are appropriately set to attain the purposes. The suspension is maintained under neutral or basic conditions, particularly under weak basic conditions, for example, at pH ranging from 5 to 10, particularly preferably 6 to 9. The temperature can be set appropriately, usually with stirring, and ranges from, for example, room temperature to 100°C, preferably 40 to 90°C. The whole amount of the Si component added could be deposited and attached onto the substrate surface where the first layer is formed, by properly selecting the conditions.

In the present invention, when micas such as muscovite and sericite are used as the thin platelet-like substrate, the coating with the Si hydrous oxide layer on the first layer can be formed easily by coating the thin platelet-like substrate with the first layer.

The amount of the Si hydrous oxide constituting the second layer is preferably 10 to 40% by weight in terms of silicon dioxide with respect to the whole weight of the filler. If the amount is smaller than this range, the semi-transparency of the present invention is difficult to obtain due to a high possibility that a third layer (Ti hydrous oxide layer) described below is directly attach/deposited onto the first layer. A too large amount of the Si hydrous oxide is not preferable because of too high transparency.

Preferably, the thin platelet-like substrate after the second layer formation has asperity on its surface. It can be confirmed as an interface between the second layer and the third layer by observing the cross section of the filler with SEM (scanning electron microscope) after the calcination of the filler. Specifically, it is preferred that the interface in the cross-sectional SEM image should have, in the range of 1 µm distance in the planar direction of the substrate, second layer's asperity where a difference between the highest and lowest heights from the substrate surface ranges from 30 nm to 200 nm inclusive. In general, the lowest and largest heights are 10 to 100 nm and 40 to 300 nm, respectively. It is further preferred that such asperity should also be formed in the range of 0.5 µm distance in the planar direction of the substrate.

The asperity on the surface of the second layer can be achieved by forming the first layer, particularly by properly setting the coating weight as described above. The asperity itself on the surface of the second layer is considered to contribute to transparency and a moderate light-scattering property and is also considered to have an influence on the formation of aggregation of crystals of the third layer (aggregates of the crystals) described below.

In the subsequent third layer-forming step, the thin platelet-like substrate comprising the second layer formed thereon is further coated with a Ti hydrous oxide.

Examples of Ti salt for forming the Ti hydrous oxide layer include salts of titanium tetrachloride and titanyl sulfate. Preferably, they are used in an aqueous solution form. An alkali compound used to deposit the Ti hydrous oxide and gradually added simultaneously with the Ti salt (aqueous solution) (preferably, by dropping) under acidic conditions is sodium hydroxide, potassium hydroxide, or the like. Preferably, they are used in an aqueous solution form.

Conditions such as pH in the suspension, a temperature, and addition time are appropriately set to attain the purposes. The suspension is maintained under acidic conditions, for example, at pH ranging from 1 to 3, particularly preferably 1.5 to 2.5. Usually, this addition is performed with stirring. The temperature can be set appropriately. Generally, it ranges from, for example, room temperature to 100°C, preferably 40 to 90°C. The whole amount of the Ti component added can be deposited and attached onto the substrate surface after the second layer formation by properly selecting the conditions.

Before the initiation of addition of the Ti salt (aqueous solution), the suspension is rendered acidic. During this procedure, very small amount of the hydrous oxide and/or carbonate of the first metal may be eluted from the first layer coated initially. The eluted metal ions of the first metal are considered to be re-deposited and re-attached onto the third layer because the pH of the suspension is finally adjusted to a neutral range after the third layer formation. Thus, the third layer is mainly composed of the Ti hydrous oxide and also encompasses a third layer comprising very small amount of the first metal.

The amount of the third layer, that is, the Ti hydrous oxide is preferably 20 to 50% by weight in terms of titanium dioxide with respect to the whole weight of the filler. A too small amount of the Ti hydrous oxide is not preferable because of too high transparency, while a too large amount thereof is not preferable because of an increased bluish tint.

The thin platelet-like substrate after the third layer formation has crystals (primary particles) of the Ti hydrous oxide on its surface, and they further gather and form aggregates (secondary particle). The aggregates can be confirmed by the SEM observation of the surface after the calcination of the filler. In the present invention, the crystals (primary particles) of the Ti hydrous oxide by the SEM image have a particle diameter of, for example, 10 nm to 70 nm, and these crystals should gather and form aggregates, preferably having the size of 70 nm to 500 nm. The aggregates are present in a flower-shaped or granular form and are preferably 100 nm to 450 nm in size, more preferably 150 nm to 400 nm in size. The aggregates of the Ti hydrous oxide are considered to prevent excessive light scattering caused by reflection, impart a moderate scattering effect, contribute to improvement in transmittance, and impart moderate transparency (semi-transparency) without a bluish tint.

Subsequently, an after-treatment step is shown below. A solid matter is separated by washing and filtration from the suspension adjusted to the neutral range. The solid matter is dried at 105°C to 150°C and then calcined, if desired, at a temperature of 500°C to 850°C. After calcination, aggregations and so on are removed with a sieve or the like.

In the present invention, the first layer is preferably composed of (i.e. essentially composed of) hydrous oxide, carbonate, or calcined product thereof, of the first metal; the second layer is preferably composed of (i.e. essentially composed of) Si hydrous oxide or calcined product thereof; and the third layer is preferably composed of (i.e. essentially composed of) Ti hydrous oxide or calcined product thereof. In the present invention, the term "hydrous oxide" representatively refers to hydroxide forms, oxide forms, and intermediate forms thereof (hydrous oxides or oxide hydrates). For example, when the calcination step is adopted, a higher temperature at the step is more apt to form the oxide. If only drying is used, the resulting product is more likely to be present in the hydroxide or hydrous oxide (oxide hydrate) form. Moreover, the "carbonate" of the first metal means that the carbonate is present as the main component. The carbonate may comprise a hydrate thereof, hydrous oxide thereof, and the like. In general, a part or the whole thereof, when undergoing a high-temperature step such as calcination, is converted into an oxide. This means that the substances constituting the first to third layers in the filler of the present invention may stand at any stage from a deposited form to a form completely converted into an oxide by calcination.

The filler of the present invention is produced by the process as described above and has perfect luminous transmittance (τt) of 70% or higher and parallel light transmittance of 20% or higher according to ISO 13468-1 (JIS K7361) as well as haze (cloudiness value) of 40 to 70 according to ISO 14782 (JIS K7136) in the measuring condition shown below.

The perfect luminous transmittance is more preferably 75% or higher. It is usually 95% or lower and is 90% or lower in a representative example. The haze value is more preferably 45 to 70, and the parallel light transmittance is more preferably 25% or higher.

In the present invention, the perfect luminous transmittance (τt), the parallel light transmittance, and the haze (cloudiness value) were measured with a "haze/transmittance meter HM-150" manufactured by Murakami Color Research Laboratory Co., Ltd., by preparing a sample for measurement shown below. The sample for measurement was prepared by adding 0.5 g of the filler obtained by the present invention to 9.5 g of vinyl chloride-base medium comprising 20% by weight of solid matters (e.g., "VS Medium" from Dainichiseika Color & Chemicals Mfg. Co., Ltd.), followed by mixing by stirring, and applying the resulting dispersion solution to a quartz glass plate of 0.15 cm thick x 5 cm wide x 5 cm long by use of a bar coater No. 20. That is to say, the filler of the present invention is characterized in that the sample prepared by such a method possesses the given optical properties. The film thickness immediately before drying is 40 to 50 µm according to this method for preparing the sample. Thus, the dried coating film including the filler by about 20% by weight has a film thickness of 7.0 to 15 µm. It is only desired that thus-obtained dried coating have the given optical properties.

In the present invention, the foggy effects refer to perfect luminous transmittance (τt) of 70% or higher, parallel light transmittance of 20% or higher, and haze (cloudiness value) of 40 to 70 according to ISO 14782 (JIS K7136) in the measuring condition shown above.

Preferably, the filler of the present invention has oil absorption ranging from 80 to 150 ml/100 g. Those produced as described above and satisfying the given optical properties generally satisfy this range of the amount of oil absorption. The filler having oil absorption falling within this range is suitable for a cosmetic, particularly for uses in a foundation.

Preferably, the filler of the present invention has a coefficient of friction (MIU value) of 0.75 or lower measured with a KES friction tester (KES-SE-DC tester from Kato Tech Co., Ltd.). Those produced as described above and satisfying the given optical properties generally satisfy this range and have favorable texture to skin. The coefficient of friction is generally 0.5 or higher. This friction coefficient can be measured by the KES-SE-DC tester by attaching a two-side adhesive tape to a slide glass, attaching the test sample there onto, and sliding a silicone sensor by 20 mm.

### [Application of filler]

The filler of the present invention can be used in a foundation and other cosmetics. Specifically, the cosmetic of the present invention includes make-up cosmetics, hair cosmetics, and antiperspirants. Specific examples thereof can include gels, lip rouges, foundations (e.g., emulsion type, liquid type, and oil type), compact cake, creams, lip sticks, blushers, mascaras, nail enamels, eyebrow colors, eye shadows, eyeliners, hair remedies, antiperspirant powders, and antiperspirant sprays. The amount of the filler of the present invention formulated can be set appropriately according to the purposes and is, for example, 1 to 50% by weight in a cosmetic. For example, the amount is exemplified by 1 to 25 wt% in a foundation, 1 to 40 wt% in an eye shadow, 1 to 20 wt% in a lip rouge, and 0.1 to 10 wt% in a nail enamel.

These cosmetics comprise the filler of the present invention as essential component and further comprises as other active ingredients, one or more kinds of members selected from skin protective agents, coloring agents and other extender pigments, sunscreens, antiperspirants, moisturizers, antimicrobial agents/bactericides, texture-improving agents, oils, and foam stabilizers.

The skin protective agents are formulated for the purpose of preventing skin roughness, and examples thereof include: liquid fats and oils such as paraffins, esters, higher alcohols, and glycerides; and acrylic, styrene-base, ether-base, ester-base, and silicone-base polymer emulsions or suspensions.

The coloring agents and other pigments include water-insoluble pigments, lipid-soluble dyes, vat dyes, and lake dyes and are specifically exemplified by the followings: titanium dioxide, calcium carbonate, clay, talc, barium sulfate, white carbon black, chromium oxide, zinc white, zinc sulfide, zinc powders, metal powder pigments, iron black, yellow iron oxide, colcothar, chrome yellow, carbon black, molybdate orange, iron blue, ultramarine blue, cadmium-base pigments, fluorescent pigments, soluble azo pigments, insoluble azo pigments, condensed azo pigments, phthalocyanine pigments, condensed polycyclic pigments, composite oxide-base pigments, graphite, mica (e.g., muscovite, phlogopite, synthetic mica, and fluorine tetrasilicon mica), metal oxide-coated micas (e.g., titanium oxide-coated mica, titanium dioxide-coated mica, (hydrous) iron oxide-coated mica, mica coated with iron oxide and titanium oxide, and lower titanium oxide-coated mica), metal oxide-coated graphite (e.g., titanium dioxide-coated graphite), thin platelet-like alumina, metal oxide-coated thin platelet-like aluminas (e.g., titanium dioxide-coated thin platelet-like alumina, iron oxide-coated thin platelet-like alumina, iron sesquioxide-coated thin platelet-like alumina, triiron tetroxide-coated thin platelet-like alumina, and metal oxide-coated thin platelet-like alumina having interference color), MIO, sericite, magnesium carbonate, silica, zeolite, hydroxyapatite, chromium oxide, cobalt titanate, glass beads, nylon beads, and silicone beads. Organic pigments are exemplified by red Nos. 2, 3, 102, 104, 105, 106, 201, 202, 203, 204, 205, 206, 207, 208, 213, 214, 215, 218, 219, 220, 221, 223, 225, 226, 227, 228, 230 (1), 230 (2), 231, 232, and 405, yellow Nos. 4, 5, 201, 202-1, 202-2, 203, 204, 205, 401, 402, 403, 404, 405, 406, and 407, green Nos. 3, 201, 202, 204, 205, 401, and 402, blue Nos. 1, 2, 201, 202, 203, 204, 205, 403, and 404, orange Nos. 201, 203, 204, 205, 206, 207, 401, 402, and 403, brown No. 201, violet Nos. 201 and 401, and black No. 401. Natural dyes include salol yellow, carthamin, β-carotene, hibiscus color, capsaicin, carminic acid, laccaic acid, curcumin, riboflavin, and shikonin.

Examples of the sunscreens include: organic compounds such as benzophenone compounds, dibenzoylmethane derivatives, and cinnamate derivatives; and inorganic compounds such as titanium oxide and zinc oxide.

Examples of the antiperspirants include aluminum hydroxychloride, tannic acid, and zinc sulfate.

Examples of the moisturizers include glycerin, glycol, sorbitol, and polyols such as polyethylene glycol.

Examples of the antimicrobial agents/bactericides include: alcohols such as ethyl alcohol and isopropyl alcohol; phenols such as phenol and ortho-phenylphenol; aldehydes such as formaldehyde and glutaraldehyde; carboxylic acids such as benzoic acid (and Na salts thereof), zinc 10-undecylenate, and octanoic acid (salt); and various thiazole-base, various peroxide-base, and various quaternary amine activator-base antimicrobial agents/bactericides.

The texture-improving agents used in the present invention can be other extender pigments or powders such as synthetic polymer particles and natural polymer particles. Examples thereof include: inorganic compounds such as talc, kaoline, sericite, calcium carbonate, magnesium silicate, and silicates; synthetic polymer particles such as nylon powders, polyethylene powders, polystyrene powders, Tetron powders, epoxy resin powders, and silicone resin powders; and natural polymer particles such as chitosan particles, starch particles, cellulose particles, silk powders, and crystalline cellulose powders.

As oils, both volatile and nonvolatile oils are used in the present invention. Examples thereof include liquid oils such as hydrocarbon oils (e.g., mineral oil), ester oils (e.g., isopropyl myristate and caprylic acid triglyceride), vegetable oils, low-viscosity silicone oils, and volatile silicone oils; solid paraffin and vaseline; ceramide, ethylene glycol difatty acid ester, and dialkyl ether; compounds having a silanol skeleton such as methylpolysiloxane, methylphenylsiloxane, and methylhydrogenpolysiloxane; and silicone resins and silicone beads.

The foam stabilizers are activators that stabilize a foam membrane and include water-soluble polymers and hydrophilic solids. The water-soluble polymers include nonionic polymers such as methyl cellulose, hydroxypropyl cellulose, polyvinyl alcohol, and polyacrylamide; anionic polymers such as sodium salts of xanthan gum and polyacrylic acid and a sodium salt of carboxymethyl cellulose; and cationic polymers such as hydroxypropyltrimethylammonium chloride guar gum and hydroxypropyltrimethylammonium chloride starch.

Perfumes are optionally used in the cosmetic of the present invention. Moreover, various sodas, soft soaps, various metallic (zinc, calcium, magnesium) soaps, various surfactants such as sorbitan fatty acid ester, and so on are optionally used.

The filler of the present invention can be used preferably in applications other than cosmetics. For example, the filler can be mixed with resins consisting of thermoplastic resins and thermosetting resins and with oils consisting of fat and oil and alcohols, and used in resin compositions or resin moldings such as bottles and toys. In this case, the filler is mixed with the resin either directly or after being incorporated in advance as a pellet. Then, the mixture can be made into various moldings by extrusion molding, calendering, blowing, or the like. Resin components for which the filler is used can be both thermoplastic and thermosetting resins such as polyolefins, epoxies, polyesters, polyamides nylons), polycarbonates, and polyacrylates.

Furthermore, the filler can be mixed with printing ink and can be used as ink with improved printability. Moreover, the filler can be mixed into paint and can be used in paint with improved paintability and coatability.

### [Examples]

Hereinafter, the present invention will be described more in more details.

### <Example 1 >

In 1750 ml of water, 113 g of muscovite having a particle diameter of 1 to 15 µm was suspended. This suspension was heated to 80°C under stirring. To this suspension, 470 g of aqueous solution containing 70 g of aluminum chloride hexahydrate dissolved in 400 ml of water and 32 wt% sodium hydroxide aqueous solution were added in dropwise at the same time pH was maintained at 9.5.

After the completion of the dropping of the aluminum chloride aqueous solution, 1600 g of sodium silicate aqueous solution (7 wt%-SiO₂) and dilute hydrochloric acid were used and dropped at the same time with pH maintained at 9.2. Subsequently, the pH of the suspension was adjusted to acidic 1.3 with dilute hydrochloric acid. To this suspension, 1000 ml of titanium tetrachloride aqueous solution (454 g/l) and 32 wt% sodium hydroxide aqueous solution were added and dropped at the same time with pH maintained at 1.6.

After the completion of the dropping of the titanium tetrachloride aqueous solution, 32 wt% sodium hydroxide aqueous solution was dropped to adjust the pH of the suspension to 5.0. After being filtrated and washed with water, the resulting product was dried at a temperature of 110°C and then calcined at 700°C to produce a filler of interest.
Based on the starting materials, the proportions by weight of the substrate and each layer to the whole filler were 26.2 % muscovite, 3.4% Al₂O₃, 26.0% SiO₂, and 44.4% TiO₂.

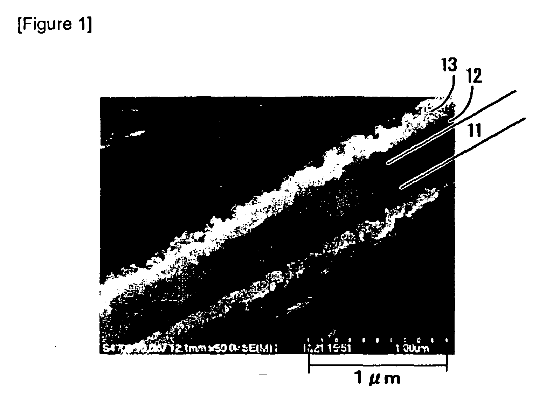

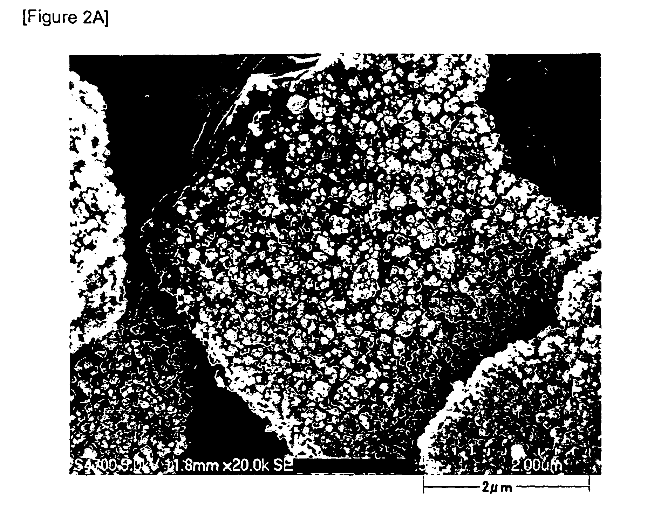

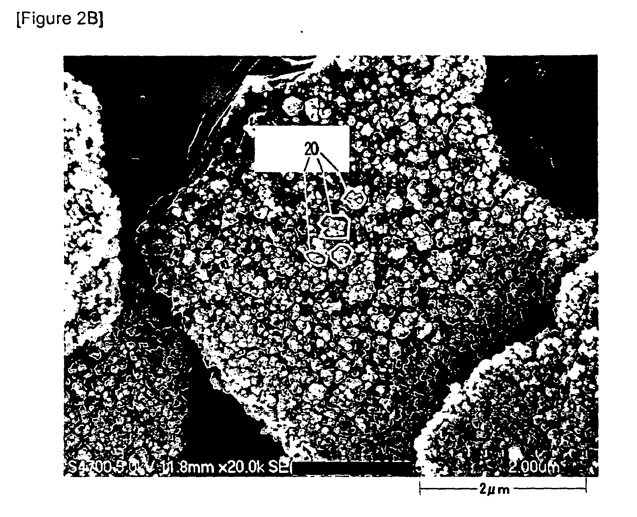

A cross-sectional SEM image of the filler is shown in Figure 1. The asperate surface of the silica layer is observed. An SEM image of the filler surface is shown in Figure 2A. Figure 2B is the same image as in Figure 2A and shows the size of a secondary aggregate of titanium dioxide crystals.

Measurement results of optical properties of the filler are shown in Table 1. Its oil absorption measured using linseed oil was 110 ml/100 g, and its average coefficient of friction (MIU value) as extensibility, glidability, and adhesion corresponding to texture to skin was 0.66.

### <Example 2>

In 1700 ml of water, 110 g of muscovite having a particle diameter of 1 to 15 µm was suspended. This suspension was heated to 80°C under stirring. To this suspension, 470 g of aqueous solution containing 70 g of aluminum chloride hexahydrate dissolved in 400 ml of water and 32 wt% sodium hydroxide aqueous solution were added dropwise at the same time while maintaining the pH at 9.5.

After the completion of the dropping of the aluminum chloride aqueous solution, 800 g of sodium silicate aqueous solution (5 wt%-SiO₂) and dilute hydrochloric acid were used and added dropwise at the same time with pH maintained at 9.2. Subsequently, the pH of the suspension was adjusted to acidic 1.3 with dilute hydrochloric acid. To this suspension, 825 ml of titanium tetrachloride aqueous solution (385 g/l) and 32 wt% sodium hydroxide aqueous solution were dropped at the same time while maintaining the pH at 1.6.

After the completion of the dropping of the titanium tetrachloride aqueous solution, 32 wt% sodium hydroxide aqueous solution was dropped to adjust the pH of the suspension to 5.0. After being filtrated and washed with water, the resulting product was dried at a temperature of 110°C and then calcined at 700°C to produce a filler of interest. As in Example 1, the silica layer had asperate, and secondary aggregates of titanium oxide crystals were present on the filler surface.

Based on the starting materials, the proportions by weight of the substrate and each layer to the whole filler were 36.8% muscovite, 5.0% Al₂O₃, 13.4% SiO₂, and 44.8% TiO₂.

Measurement results of optical properties of the filler are shown in Table 1. Its oil absorption measured using linseed oil was 135 ml/100 g, and its MIU value was 0.68.

### <Comparative Example 1>

A filler was prepared under the conditions of Example 1 without use of a metal salt of Al corresponding to the first metal. A coating layer of an Si hydrous oxide layer was not observed.

### <Comparative Example 2>

Extender-W (product commercially available form Merck Ltd., Japan, 50% by weight of titanium oxide-coated mica, based on Japanese Patent No. 2121498) was prepared as a conventional filler. Measurement results of optical properties of the filler are shown in Table 1.

**[Table 11**

| Sample | Perfect luminous transmittance (%) (τt) | Parallel light transmittance (%) | Haze (cloudiness value) |
|---|---|---|---|
| Example 1 | 79.8 | 36.1 | 54.8 |
| Example 2 | 75.4 | 25.6 | 66.0 |
| Comparative Example 2^{*)} | 69.8 | 16.2 | 76.8 |

| | | | |
|---|---|---|---|
| ^{*)} Extender-W: product commercially available from Merck Ltd., Japan (based on Japanese Patent No. 2121498), 50% by weight of titanium oxide-coated mica | | | |

### <Formulation example in foundation>

A foundation was prepared according to the following formulation:
38 parts by weight of talc
10 parts by weight of filler of Example 1
10 parts by weight of mica (8 µm)
3 parts by weight of magnesium stearate
8 parts by weight of nylon-12 powder
1.9 parts by weight of yellow iron oxide
0.8 parts by weight of red iron oxide
1.0 part by weight of titanium oxide

Appropriate amount of mineral oil
3.3 parts by weight of (caprylic acid, capric acid)triglyceride
0.1 parts by weight of butylparaben

### <Formulation example in compact powder>

A compact powder was prepared according to the following formulation:
50 parts by weight of talc
10 parts by weight of filler of Example 1
5 parts by weight of color pigment

Appropriate amount of isopropyl myristate
2 parts by weight of magnesium stearate

### [Industrial Applicability]

A filler of the present invention has foggy effects, smooth glidability over skin, excellent adherence to skin, favorable texture to skin, and moderate oil absorption. It can be used preferably for a cosmetic, particularly for uses in a foundation. Furthermore, the filler of the present invention can also be used as a filler for a variety of other applications.

### [Brief Description of the Drawings]

[Figure 1]
   Figure 1 is a cross-sectional SEM image of the filler produced in Example 1. Aluminum oxide (aluminum hydrous oxide) corresponding to the first layer is not seen.
[Figure 2A]
   Figure 2A is an SEM image of the surface of the filler produced in Example 1.
[Figure 2B]
   Figure 2B is a diagram showing an aggregate of titanium oxide crystals in the SEM image of Figure 2A. In the diagram, a representative example of the aggregates are indicated by enclosing them by polygons slightly larger than the aggregates.

### [Description of Symbols]

11 Substrate
12 Silica layer
13 Titanium oxide layer
14 Aggregate of titanium oxide crystals

## Claims

1. A filler comprising:
a transparent or semitransparent thin platelet-like substrate;
a particle layer or coating layer (hereinafter, referred to as a first layer) which is formed on the surface of the thin platelet-like substrate and contains a hydrous oxide, carbonate, or calcined product thereof, of at least one metal (hereinafter, referred to as a first metal) selected from the group consisting of Mg, Al, and Ca;
a coating layer (hereinafter, referred to as a second layer) which coats the thin platelet-like substrate comprising the first layer formed thereon and contains an Si hydrous oxide or calcined product thereof; and a coating layer (hereinafter, referred to as a third layer) which coats the second layer and contains a Ti hydrous oxide or calcined product thereof.

2. A filler according to claim 1, wherein the thin platelet-like substrate has a shape with an aspect ratio of 10 to 100 represented by average particle diameter/average thickness and an average particle diameter of 5 to 20 µm.

3. A filler according to claim 1 or 2, wherein the thin platelet like substrate is selected from the group consisting of, natural and synthetic mica, thin platelet-like alumina, talc, glass flake, kaolin and thin platelet-like silica.

4. A filler according to any of claims 1 to 3, wherein an interface between the second layer and the third layer has, in a range of 1 µm distance, the asperity of second layer where a difference between the highest and lowest heights from the substrate surface ranges from 30 nm to 200 nm.

5. A filler according to any of claims 1 to 4, wherein the filler comprises aggregates of 70 nm to 500 nm formed of crystals of the Ti hydrous oxide or calcined product thereof on the surface.

6. A filler according to any of claims 1 to 5, wherein the proportion by weight of each layer to the whole filler is
1 to 7% by weight of the first layer in terms of a metallic oxide of the first metal,
10 to 40% by weight of the second layer in terms of silicon dioxide, and
20 to 50% by weight of the third layer in terms of titanium dioxide.

7. A filler according to any of claims 1 to 6, wherein the filler has oil absorption ranging from 80 to 150 ml/100 g.

8. A filler according to any of claims 1 to 7, wherein the filler has a friction coefficient (MIU value) of 0.75 or lower measured with a KES friction tester.

9. A dried coating film **characterized in that** it includes 20 % by weight of the filler according to any of claims 1 to 8 and having a film thickness ranging from 7.0 to 15 µm exhibits perfect luminous transmittance (τt) of 70% or higher, parallel light transmittance of 20% or higher each according to ISO 13468-1 (JIS K7361), and haze of 40 to 70 according to ISO 14782 (JIS K7136).

10. A process for producing a filler according to any of claims 1 to 8, comprising:
a first layer-forming step of suspending a transparent or semitransparent thin platelet-like substrate in water and gradually adding to this suspension under neutral or basic conditions, an aqueous solution of a salt of at least one metal (hereinafter, referred to as a first metal) selected from the group consisting of Mg, Al, and Ca and an alkali aqueous solution or carbonate aqueous solution, thereby depositing a hydrous oxide and/or carbonate of the first metal in a particle or layer form onto the surface of the thin platelet-like substrate;
a second layer-forming step of gradually adding an alkali aqueous solution of silicic acid and dilute mineral acid at the same time under neutral or basic conditions, thereby coating with an Si hydrous oxide, the thin platelet-like substrate comprising the first layer formed thereon; and
a third layer-forming step of gradually adding a Ti salt aqueous solution and an alkali aqueous solution at the same time under acidic conditions, thereby coating with a Ti hydrous oxide, the thin platelet-like substrate comprising the second layer formed thereon.

11. A process according to claim 10, further comprising a step of calcining the thin platelet-like substrate after the formation of the third layer.

12. Use of the filler according to any of claims 1 to 8 in cosmetics, lacquers, plastics, inks, printing inks and coatings.

13. A cosmetic containing 1-50 wt.% based on the cosmetic formulation of a filler according to any of claims 1 to 8.

## Patentansprüche

1. Füllstoff, enthaltend
ein transparentes oder semitransparentes dünnes plättchenartiges Substrat;
eine Partikel- oder Beschichtungsschicht (im Folgenden als erste Schicht bezeichnet), die auf der Oberfläche des dünnen plättchenartigen Substrats gebildet ist und ein wasserhaltiges Oxid, Carbonat oder kalziniertes Produkt davon von mindestens einem Metall (im Folgenden als erstes Metall bezeichnet) enthält, das aus der Gruppe Mg, Al und Ca ausgewählt ist;
eine Beschichtungsschicht (im Folgenden als zweite Schicht bezeichnet), die das dünne plättchenartige Substrat mit der darauf gebildeten ersten Schicht bedeckt und ein wasserhaltiges Si-Oxid oder kalziniertes Produkt davon enthält;
und eine Beschichtungsschicht (im Folgenden als dritte Schicht bezeichnet), die die zweite Schicht bedeckt und ein wasserhaltiges Ti-Oxid oder kalziniertes Produkt davon enthält.

2. Füllstoff nach Anspruch 1, bei dem das dünne plättchenartige Substrat eine Form mit einem Aspect Ratio von 10 zu 100, dargestellt durch einen durchschnittlichen Teilchendurchmesser / durchschnittliche Dicke, und einen durchschnittlichen Teilchendurchmesser von 5 bis 20 µm, aufweist.

3. Füllstoff nach Anspruch 1 oder 2, bei dem das dünne plättchenartige Substrat aus der Gruppe natürlicher und synthetischer Glimmer, dünnes plättchenartiges Aluminiumoxid, Talkum, Glasplättchen, Kaolin und dünnes plättchenartiges Siliciumdioxid ausgewählt ist.

4. Füllstoff nach einem beliebigen der Ansprüche 1 bis 3, wobei zwischen der zweiten Schicht und der dritten Schicht eine Berührungsfläche in einem Bereich von 1 µm Abstand ist, und die Oberflächenunebenheit der zweiten Schicht zwischen der höchsten und der niedrigsten Unebenheit von der Substratoberfläche eine Differenz im Bereich von 30 nm bis 200 nm aufweist.

5. Füllstoff nach einem beliebigen der Ansprüche 1 bis 4, bei dem der Füllstoff Aggregate von 70 nm bis 500 nm enthält, die aus Kristallen des wasserhaltigen Ti-Oxids oder des kalzinierten Produktes davon an der Oberfläche gebildet werden.

6. Füllstoff nach einem beliebigen der Ansprüche 1 bis 5, bei dem der Gewichtsanteil jeder einzelnen Schicht am gesamten Füllstoff
1 bis 7 Gew.-% erste Schicht, ausgedrückt als Metalloxid des ersten Metalls,
10 bis 40 Gew.-% zweite Schicht, ausgedrückt als Siliciumdioxid, und
20 bis 50 Gew.-% dritte Schicht, ausgedrückt als Titandioxid,
beträgt.

7. Füllstoff nach einem beliebigen der Ansprüche 1 bis 6, bei dem der Füllstoff eine Ölabsorption im Bereich von 80 bis 150 ml/100 g aufweist.

8. Füllstoff nach einem beliebigen der Ansprüche 1 bis 7, bei dem der Füllstoff einen Reibungskoeffizienten (MIU-Wert) von 0,75 oder weniger, gemessen mit einem KES-Reibungsprüfgerät, aufweist.

9. Getrockneter Beschichtungsfilm, **dadurch gekennzeichnet, dass** er 20 Gew.-% des Füllstoffs nach einem beliebigen der Ansprüche 1 bis 8 umfasst und bei einer Filmdicke im Bereich von 7,0 bis 15 µm eine perfekte Lichtdurchlässigkeit (τt) von 70% oder mehr, eine parallele Lichtdurchlässigkeit von 20% oder mehr, jeweils nach ISO 13468-1 (JIS K7361), und eine Trübung von 40 bis 70 nach ISO 14782 (JIS K7136) zeigt.

10. Verfahren zur Herstellung eines Füllstoffs nach einem beliebigen der Ansprüche 1 bis 8, mit
einem ersten schichtbildenden Schritt, in dem man ein transparentes oder semitransparentes dünnes plättchenartiges Substrat in Wasser suspendiert und diese Suspension unter neutralen oder basischen Bedingungen langsam mit einer wässerigen Lösung eines Salzes von mindestens einem Metall (im Folgenden als erstes Metall bezeichnet), das aus der Gruppe Mg, Al und Ca ausgewählt ist, und einer wässrigen Alkalilösung oder wässrigen Carbonatlösung versetzt, wodurch ein wasserhaltiges Oxid und/oder Carbonat des ersten Metalls in Teilchen- oder Schichtform auf der Oberfläche des dünnen plättchenartigen Substrats abgeschieden wird;
einem zweiten schichtbildenden Schritt, in dem man gleichzeitig unter neutralen oder basischen Bedingungen langsam eine wässrige Alkalilösung von Kieselsäure und verdünnte Mineralsäure zugibt, wodurch das dünne plättchenartige Substrat mit der darauf gebildeten ersten Schicht mit einem wasserhaltigen Si-Oxid bedeckt wird, und
einem dritten schichtbildenden Schritt, bei dem man gleichzeitig unter sauren Bedingungen langsam eine wässrige Ti-Salzlösung und eine wässrige Alkalilösung zugibt, wodurch das dünne plättchenartige Substrat mit der darauf gebildeten zweiten Schicht mit einem wasserhaltigen Ti-Oxid bedeckt wird.

11. Verfahren nach Anspruch 10, welches als weiteren Schritt die Kalzinierung des dünnen plättchenartigen Substrats beinhaltet, nach der Aufbringung der dritten Schicht.

12. Verwendung des Füllstoffs nach einem beliebigen der Ansprüche 1 bis 8 in Kosmetika, Lacken, Kunstoffen, Tinten, Druckfarben und Beschichtungen.

13. Kosmetikum, das 1-50 Gew.-% eines Füllstoffs nach einem beliebigen der Ansprüche 1 bis 8, bezogen auf die kosmetische Zubereitung, enthält.

## Revendications

1. Charge comprenant :
un substrat fin semblable à des plaquettes, transparent ou semi-transparent ;
une couche de particule ou une couche de revêtement (désignée dans ce qui suit par « première couche ») qui est formée à la surface du substrat fin semblable à des plaquettes, et qui contient un carbonate, oxyde hydraté ou produit calciné de ceux-ci, d'au moins un métal (désigné dans ce qui suit par « premier métal ») choisi dans le groupe constitué par Mg, Al et Ca ;
une couche de revêtement (désignée dans ce qui suit par « deuxième couche ») qui revêt le substrat fin semblable à des plaquettes comprenant la première couche y étant formée, et qui contient un oxyde hydraté de Si, ou un produit calciné de celui-ci ;
et une couche de revêtement (désignée dans ce qui suit par « troisième couche ») qui revêt la deuxième couche et qui contient un oxyde hydraté de Ti, ou un produit calciné de celui-ci.

2. Charge selon la revendication 1, dans laquelle le substrat fin semblable à des plaquettes possède une forme ayant un rapport de longueur allant de 10 à 100 représenté par un diamètre de particules moyen/épaisseur moyenne et un diamètre de particules moyen allant de 5 à 20 µm.

3. Charge selon la revendication 1 ou 2, dans laquelle le substrat fin semblable à des plaquettes est choisi dans le groupe constitué par le mica naturel et synthétique, l'alumine fine semblable à des plaquettes, le talc, les paillettes de verre, le kaolin et la silice fine semblable à des plaquettes.

4. Charge selon l'une quelconque des revendications 1 à 3, dans laquelle l'interface entre la deuxième couche et la troisième couche possède, dans une plage de distance de 1 µm, l'aspérité d'une deuxième couche où une différence entre les hauteurs les plus élevées et les plus faibles de la surface du substrat va de 30 nm à 200 nm.

5. Charge selon l'une quelconque des revendications 1 à 4, dans laquelle la charge comprend des agrégats allant de 70 nm à 500 nm formés de cristaux de l'oxyde hydraté de Ti, ou d'un produit calciné de celui-ci, à la surface.

6. Charge selon l'une quelconque des revendications 1 à 5, dans laquelle la proportion en poids de chaque couche par rapport à la charge entière est
de 1 à 7% en poids de la première couche en termes d'oxyde métallique du premier métal,
de 10 à 40% en poids de la deuxième couche en termes de dioxyde de silicium, et
de 20 à 50% en poids de la troisième couche en termes de dioxyde de titane.

7. Charge selon l'une quelconque des revendications 1 à 6, dans laquelle la charge possède une absorption d'huile allant de 80 à 150 ml/100 g.

8. Charge selon l'une quelconque des revendications 1 à 7, dans laquelle la charge possède un coefficient de friction (valeur MIU) de 0,75 ou moins, mesuré à l'aide d'un testeur de friction KES.

9. Film de revêtement séché, **caractérisé en ce qu'**il comporte 20% en poids de la charge selon l'une quelconque des revendications 1 à 8, et ayant une épaisseur de film allant de 7,0 à 15 µm, présente un facteur de transmission lumineuse parfait (τₜ) de 70% ou plus, un facteur de transmission de lumière parallèle de 20% ou plus, chacun selon ISO 13468-1 (JIS K7361), et un trouble allant de 40 à 70 selon ISO 14782 (JIS K7136).

10. Procédé de production d'une charge selon l'une quelconque des revendications 1 à 8, comprenant :
une étape de formation d'une première couche par mise en suspension d'un substrat fin semblable à des plaquettes, transparent ou semi-transparent, dans de l'eau et par ajout graduel à cette suspension, dans des conditions neutres ou basiques, d'une solution aqueuse d'un sel d'au moins un métal (désigné dans ce qui suit par « premier métal ») choisi dans le groupe constitué par Mg, Al et Ca, et d'une solution aqueuse alcaline ou d'une solution aqueuse de carbonate, déposant ainsi un carbonate et/ou oxyde hydraté du premier métal sous forme de particule ou de couche à la surface du substrat fin semblable à des plaquettes ;
une étape de formation d'une deuxième couche par ajout graduel d'une solution aqueuse alcaline d'acide silicique et d'acide minéral dilué en même temps dans des conditions neutres ou basiques, revêtant ainsi par un oxyde hydraté de Si le substrat fin semblable à des plaquettes comprenant la première couche y étant formée ; et
une étape de formation d'une troisième couche par ajout graduel d'une solution aqueuse d'un sel de Ti et d'une solution aqueuse alcaline en même temps dans des conditions acides, revêtant ainsi par un oxyde hydraté de Ti le substrat fin semblable à des plaquettes comprenant la deuxième couche y étant formée.

11. Procédé selon la revendication 10, comprenant en outre une étape consistant à calciner le substrat fin semblable à des plaquettes après formation de la troisième couche.

12. Utilisation de la charge selon l'une quelconque des revendications 1 à 8 dans des produits cosmétiques, des laques, des matières plastiques, des encres, des encres d'impression et des revêtements.

13. Produit cosmétique contenant 1-50% en poids, sur la base de la formulation cosmétique, d'une charge selon l'une quelconque des revendications 1 à 8.
